Europäisches Patentamt

European Patent Office

Office européen des brevets

(11) Publication number: **0 510 211 A1**

# EUROPEAN PATENT APPLICATION
## published in accordance with Art. 158(3) EPC

(21) Application number: 91919171.8

(22) Date of filing: **07.11.91**

(86) International application number:
**PCT/JP91/01531**

(87) International publication number:
**WO 92/08438 (29.05.92 92/12)**

(51) Int. Cl.5: **A61K 6/06**

(30) Priority: **07.11.90 JP 301573/90**

(43) Date of publication of application:
**28.10.92 Bulletin 92/44**

(84) Designated Contracting States:
**BE CH DE FR GB LI**

(71) Applicant: **JAPAN INSTITUTE OF ADVANCED DENTISTRY**
2-1-47, Kanda Surugadai Chiyoda-ku
Tokyo 101(JP)
Applicant: **NORITAKE CO., LIMITED**
1-36, Noritake Shinmachi 3-chome
Nishi-ku Nagoya-shi Aichi 451(JP)
Applicant: **NIPPON OIL AND FATS COMPANY, LIMITED**
10-1, Yuraku-cho 1-chome
Chiyoda-ku Tokyo 100(JP)

(72) Inventor: **MASUHARA, Eiichi, 5-10,**
**Honkomagome 2-chome**
**Bunkyo-ku**
**Tokyo 113(JP)**
Inventor: **KOMIYA, Shigeo, 7-10, Minamiurawa**
**3-chome**
Urawa-shi
Saitama-ken 336(JP)
Inventor: **SAEGUSA, Takeo, 7, Fujimi-cho**
**4-chome Naka-ku**
Nagoya-shi
Aichi-ken 460(JP)
Inventor: **KOJIMA, Yasuhiro, 1-17, Aza-renda**
**Totsuka, Yamato-cho Ichinomiya-shi**
Aichi-ken 491(JP)
Inventor: **NAKAMURA, Tetsuya, 24-5, Umezono**
**2-chome**
Tsukuba-shi
Ibaragi-ken 305(JP)
Inventor: **GOTO, Yoshitaka, 24-5, Umezono**
**2-chome**
Tsukuba-shi
Ibaragi-ken 305(JP)
Inventor: **NAKAYAMA, Masaharu, 1132-9,**
**Nagakuni**
Tsuchiura-shi
Ibaragi-ken 330(JP)

(74) Representative: **Woods, Geoffrey Corlett**
**J.A. KEMP & CO. 14 South Square Gray's Inn**
**London WC1R 5LX(GB)**

(54) **PASTE FOR DENTAL GLASS IONOMER CEMENT.**

(57) A paste for dental glass ionomer cement, containing powdered glass, a water-soluble polymer and water, and a kit comprising said paste and an aqueous solution of polycarboxylic acid. Since the powdered glass is present in pasty form, the paste is advantageous in that: 1) the paste can be accurately weighed because a constant rate discharge device is available, 2) the paste can be kneaded with the polymer solution very readily in a short time, so that a sufficient treatment time is available, 3) a homogeneous mixture is available because there occurs no aggregation of the powder due to moisture absorption, and 4) the treatment room can be kept clean because the powdered glass does not scatter during the treatment.

[Technical Field]

The present invention relates to a paste for a dental glass ionomer cement and a kit comprising the paste and an aqueous solution of a polycarboxylic acid.

[Background Art]

Since dental glass ionomer cement has excellent properties such as giving no damages to dental pulp and having more preferred adhesion to dentine than zinc phosphate cement, it has been used in a wide range of clinical application such as adhesion, repair, filling and cavity lining.

The glass ionomer cement is a reaction-binding product of a silicate glass powder containing an alkaline earth metal and an aqueous solution of a polycarboxylic acid.

The glass powder is an alumino fluoro silicate glass comprising $SiO_2$, $Al_2O_3$, $CaF_2$, $BaF_2$, $AlF_3$, $AlPO_4$, etc. as a main ingredient, which is a fine powder formed by melting at 1100 - 1500°C, quenching and then pulverizing them. On the other hand, the aqueous solution containing the polycarboxylic acid comprises an aqueous solution of a polyacrylic acid or a copolymer of an acrylic acid with an itaconic acid or maleic acid, to which a tartaric acid is added.

Heretofore, when the glass ionomer cement is prepared, there has been adopted a method of scooping a predetermined amount of a glass powder by a regular spoon from a glass vessel onto a sheet of paper for kneading, while dripping a predetermined amount of an aqueous solution of a polymer onto the kneading paper sheet and sufficiently kneading them by using a spatula.

However, the prior method of kneading the powder and the liquid described above involves problems as shown below, which are desired to be dissolved.

(1) In a present method of measuring a powder, the glass powder scooped on a spoon is cut with an edge of a glass container. However, since the glass powder contains a great amount of air, it is difficult to always take a constant amount.

(2) Since the glass powder is less immiscible with the aqueous solution of a polycarboxylic acid, it tends to scatter at an initial stage of kneading, making it difficult to obtain an aimed property without a skilled technique.

(3) Since the glass powder used for the cement is generally formed as a fine powder, its surface is highly active and tends to coagulate by absorbing moisture in air. If such a glass powder is used, the performance of the cement will be deteriorated and in an extreme case, it will stick to a container to be no more usable. Accordingly, a much care has to be taken for storing the powder.

(4) Further, since the glass powder is a fine powder, when it is taken out with a predetermined amount from the container upon use, there may be a trouble, that the powder is scattered by wind at the inside or the outside of a chamber.

[Disclosure of Invention]

As a result of an earnest study for dissolving the above problems, it has been found that a glass ionomer cement composition capable of dissolving the foregoing problems, which can be obtained by combining a paste containing a glass powder, a water soluble polymer and water with an aqueous solution of a polycarboxylic acid. Thus, the present invention has been accomplished.

That is, the present invention provides a paste for a dental glass ionomer cement, comprising a glass powder, a water soluble polymer and water, as well as a kit for preparing the dental glass ionomer cement comprising the paste and the aqueous solution of the polycarboxylic acid.

As the glass powder, any conventional fine alumino fluoro silicate glass powder described above, which is usually used for dental glass ionomer cements, may be used. A specific surface area of the glass powder is usually within a range of from 2.0 to 4.0 $m^2/g$.

As the water soluble polymer, there may be mentioned cellulose derivatives such as methyl cellulose, hydroxypropylmethyl cellulose, hydroxymethylethyl cellulose, hydroxyethyl cellulose and carboxymethyl cellulose, polyvinyl alcohol and polyethylene glycol.

Further, the water soluble polymer may be used alone or as a mixture of two or more of them.

A preferred content of each of the ingredients in the paste is within a range of from 55 to 90 % by weight of the glass powder, from 9.9 to 44.9% by weight of the water and from 0.1 to 2.0% by weight of the water soluble polymer.

A paste which is stable for a long period of time can be obtained by mixing the water soluble polymer and the glass powder described above.

As the polycarboxylic acid, there may be used, for example, a polyacrylic acid, a copolymer of acrylic acid and itaconic acid and a copolymer of acrylic acid and maleic acid.

The concentration of the aqueous solution of the polycarboxylic acid is preferably within a range of from 40 to 75% by weight. The pulverization resistance of the cured glass ionomer cement will be reduced if the concentration is less than 40% by weight and, on the other hand, the viscosity of the liquid will be increased so as to worsen handlability thereof if it exceeds 75% by weight, which is not preferred.

The aqueous solution of the polycarboxylic acid can be prepared by a known method.

A preferred molecular weight of the polycarboxylic acid is generally within a range of from 1,000 to 500,000 and more preferably, within a range of from 2,000 to 100,000.

To the aqueous solution of the polycarboxylic acid, an organic acid such as tartaric acid, citric acid and maleic acid may be added by from 0.1 to 50% by weight based on the polymer.

For preparing the paste according to the present invention, an aqueous solution of a water soluble polymer is at first prepared and the solution and the glass powder are sufficiently mixed in a mixer into a homogeneous state of paste.

As the kneading method, any method not causing coagulation products with poor dispersion of the glass powder may be used and a static mixer or an ultrasonic homogenizer may be used preferably.

It is preferred to add a dispersant in the paste for maintaining the homogeneous dispersion of the glass powder over a long period of time. As the dispersant, there may be used, for example, polyethylene glycol, polyethylene glycol water soluble wax, a polymer-type anionic active agent, a polymer-type nonionic active agent, an ester-type nonionic type active agent and an ether-type nonionic active agent. However, it is preferred that the addition amount of the dispersant is less than 0.5% by weight. If the amount is greater than 0.5% by weight, collapse rate of the glass ionomer cement will increased, which is not preferred.

A sterilizer such as a cationic active agent (benzyl type) for further improving the storage stability of the paste and various kinds of adjuvants used in prior dental glass ionomer cements may be added. The addition amount of the sterilizer is preferably less than 0.5% by weight.

Viscosity of the paste is preferably almost as hard as a dentifrice paste. If it is lower, the powder and liquid may possibly be separated during a long time storage, and an amount of a curing liquid to be subsequently added will become insufficient for properly keeping the viscosity of a cement sludge, the physical property of the cement may be deteriorated. On the other hand, if the viscosity is too high, much labour will be required for taking out the kneaded product and be impractical. When such a paste is used, being filled in a constant-amount discharging device (syringe), it may facilitate a measurement for a constant amount and is convenient also for storage.

[Best Mode for Carrying Out the Invention]

The present invention will now be described more in detail with reference to examples.

Example 1

After dissolving 838 g of $SiO_2$, 572 g of $Al_2O_3$, 314 g of $CaF_2$, 186 g of NaF, 32 g of $AlF_3$ and 76 g of $AlPO_4$ at 1300 - 1400°C, they were rapidly cooled and finely pulverized by a dry process to prepare a fine alumina fluoro silicate glass powder with a specific surface area of 2.4 $m^2/g$.

A paste according to the present invention was prepared by sufficiently mixing 1000 g of the resultant fine glass powder and 300 g of an aqueous 2 wt% solution of methyl cellulose in a static mixer.

After charging 420 g of an acrylic acid, 40 g of an itaconic acid, 80 g of a tartaric acid, 25 g of ammonium persulfate and 1700 g of a distilled water in a reaction vessel and stirring at 80°C for 7 hours, they were concentrated in an evaporator to prepare a viscous aqueous solution of a polycarboxylic acid with a solid content of 70% by weight.

The paste and the aqueous solution of the polycarboxylic acid described above were respectively charged in separate polypropylene syringes and tightly capped so as not to be in contact with air.

The paste and the aqueous solution of the polycarboxylic acid were discharged at a weight ratio of 2.5 : 1 on a kneading paper and kneaded for 30 sec. Since a coagulation time was 5 min and 30 sec, which was one-thirds as short as that of conventional commercially available glass ionomer cements, a substantial operation time could be more increased than with the case of prior products and the operation for adhesion of crown was extremely facilitated. Further, since the glass powder was in the form of a paste, the glass powder did not scatter by wind during kneading.

Further, when both of them were left each in a syringe for 180 days in a room at a relative humidity of 60% and a temperature of 25°C, no change was observed.

Examples 2 - 4

The paste according to the present invention was prepared in the same manner as in Example 1 using the ingredients shown in the following Table, and kneaded with an aqueous solution of a carboxylic acid. The aqueous solution of the polycarboxylic acid used in each of Examples 2 to 4 was prepared by the method to be described later. The coagulation time and the operable time are as shown in the Table. Each of them was excellent in the kneading property. Each of the cements needed only about 30 sec for the kneading operation and could be operated with a sufficient margin. Further, they were excellent also in view of the storage stability like the paste prepared in Example 1.

On the other hand, in all of the commercially available glass ionomer cements, a part of the glass powder was coagulated to form grits under the same conditions.
(Aqueous Solution of Polycarboxylic acid used in Example 2)

Synthesis of Acrylic Acid - Itaconic Acid Copolymer

800 g of an acrylic acid, 200 g of an itaconic acid, 250 g of an isopropanol and 1010 g of a distilled water were prepared as a dripping bath 1, while 47 g of an ammonium persulfate and 575 g of a distilled water were prepared as a dripping bath 2. 1900 g of a distilled water were charged in a reaction vessel, and nitrogen substitution was conducted for 30 min. The reaction vessel was warmed to 80°C and, under a nitrogen gas stream, the dripping bath 1 and the dripping bath 2 were dripped simultaneously for about two hours. After the completion of the dripping, it was kept for further two hours and, subsequently, a temperature was elevated and reflux was conducted for three hours. After the reaction was over, it was purified by steam distillation and formed into an aqueous solution of about 65% by adjusting the concentration and then 180 g of tartaric acid was added. A molecular weight of the resultant polymer converted as polystyrene was 56,800 according to gel permeation chromatography (GPC).
(Aqueous Solution of Polycarboxylc acid used in Example 3)

Synthesis of Acrylic Acid Polymer

800 g of an acrylic acid, 480 g of an isopropyl alcohol.and 800 g of a distilled water were prepared as a dipping bath 1, while 19 g of an ammonium persulfate and 460 g a of distilled water were prepared as a dripping bath 2. 1520 g of a distilled water and 19 g of an ammonium persulfate were charged in a reaction vessel, and nitrogen substitution was conducted for 30 min. The reaction vessel was warmed to 75°C and, under a nitrogen gas stream, the dripping bath 1 and the dripping bath 2 were dripped simultaneously for about two hours. After the completion of the dripping, it was kept for further two hours and, subsequently, a temperature was elevated and reflux was conducted for three hours. After the reaction was over, it was purified by steam distillation and formed into an aqueous solution of about 65% by adjusting the concentration, and then 180 g of a tartaric acid was added. The molecular weight of the resultant polymer converted as polystyrene was 62,500 according to gel permeation chromatography (GPC). (Aqueous Solution of Polycarboxylc acid used in Example 4)

Synthesis of Acrylic Acid - Maleic Acid Copolymer

800 g of an acrylic acid, 50 g of a maleic acid, 300 g of an isopropyl alcohol and 1000 g of a distilled water were prepared as a dipping bath 1, while 50 g of an ammonium persulfate and 600 g of a distilled water were prepared as a dripping bath 2. 2000 g of a distilled water were charged in a reaction vessel, and nitrogen substitution was conducted for 30 min. The reaction vessel was warmed to 80°C and, under a nitrogen gas stream, the dripping bath 1 and the dripping bath 2 were dripped simultaneously for about two hours. After the completion of the dripping, it was kept for further two hours and, subsequently, a temperature was elevated and reflux was conducted for three hours. After the reaction was over, it was purified by steam distillation and formed into an aqueous solution of about 65% by adjusting the concentration, and then 180 g of a tartaric acid was added. The molecular weight of the resultant polymer converted as polystyrene was 53,700 according to gel permeation chromatography (GPC).

Comparative Example

A powder and solution of a commercially available glass ionomer cement "HIGH BOND TYPE II" (manufactured by Shofusha) were measured by an appended measuring device and kneaded on a sheet of paper for kneading. The powder and solution were less fit and difficult to be kneaded at an initial stage of the kneading. Further, they were difficult to be used unless an operator is sufficiently skilled for the kneading operation since the operable time was as short as 1 min 20 sec.

Table

| | Specific surface area of glass powder m$^2$/g) | Water soluble polymer | Coagulation time | Opearble time | Kneading property |
|---|---|---|---|---|---|
| Example 1 | 2.4 (100 pbw) | Methyl cellulose (aq 2 wt% solution, 30 pbw) | 5 min 30 sec | 2 min 30 sec | good |
| Example 2 | 3.4 (100 pbw) | Hydroxymethyl cellulose (aq 3 wt% solution, 80 pbw) | 4 min 30 sec | 2 min | good |
| Example 3 | 2.2 (100 pbw) | Carboxymethyl cellulose (aq 1 wt% solution, 20 pbw) | 7 min | 2 min 40 sec | good |
| Example 4 | 3.0 (100 pbw) | Polyvinyl alcohol (aq 5 wt% solution, 50 pbw) | 6 min 30 sec | 2 min 20 sec | good |
| Comp. Example | - | - | 5 min 20 sec. | 1 min 20 sec | somewhat poor |

[Industrial Applicability]

1. Since the glass powder is in a paste form, the constant amount discharging device can be utilized, thereby enabling an accurate measurement.
2. Since the glass paste is in a paste form, kneading with an aqueous polymer solution is extremely simple and since kneading is completed in a short period of time a sufficient operation time can be obtained.
3. Since the glass powder is in a paste form, it is free from a poorly homogenized state such as powder coagulation caused by moisture absorption.
4. The glass powder does not scatter during operation and a clinic room can always be kept clean.

**Claims**

1. A paste for a dental glass ionomer cement comprising a glass powder, a water soluble polymer and water.

2. A paste for a dental glass ionomer cement as defined in claim 1, wherein the content of the glass powder is of from 55 to 90% by weight, the content of the water is of from 9.9 to 44.9% by weight and the content of the water soluble polymer is of from 0.1 to 2.0% by weight.

3. A kit for preparing a dental glass ionomer cement comprising the paste as defined in claim 1 or 2 and an aqueous solution of a polycarboxylic acid.

4. A kit for preparing the dental glass ionomer cement as defined in claim 3, wherein the concentration of the polymer in the aqueous solution of the polycarboxylic acid is from 40 to 75% by weight.

5. A kit for preparing the dental glass ionomer cement as defined in claim 3 or 4, wherein the polycarboxylic acid is a polyacrylic acid, a copolymer of an acrylic acid and itaconic acid or a copolymer of an acrylic acid and maleic acid.

# INTERNATIONAL SEARCH REPORT

International Application No PCT/JP91/01531

## I. CLASSIFICATION OF SUBJECT MATTER (if several classification symbols apply, indicate all) [6]

According to International Patent Classification (IPC) or to both National Classification and IPC

Int. Cl$^5$ A61K6/06

## II. FIELDS SEARCHED

| Minimum Documentation Searched [7] | |
|---|---|
| Classification System | Classification Symbols |
| IPC | A61K6/06 |

Documentation Searched other than Minimum Documentation
to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| Y | JP, A, 51-66314 (National Research Development Corp.), June 8, 1976 (08. 06. 76), & DE, A, 2547744 & GB, A, 1532954 | 1-5 |
| Y | JP, A, 57-134407 (Haruyuki Kawahara), August 19, 1982 (19. 08. 82), Claim 5 (Family: none) | 1-5 |
| Y | JP, A, 58-99406 (Tokuyama Soda Co., Ltd.), June 13, 1983 (13. 06. 83), (Family: none) | 1-5 |
| Y | JP, A, 59-10872 (ESPE Fabrik Pharmazeutischer Praparte GmbH), January 20, 1984 (20. 01. 84), & EP, A, 23013 & US, A, 4376835 | 1-5 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| January 9, 1992 (09. 01. 92) | January 28, 1992 (28. 01. 92) |

| International Searching Authority | Signature of Authorized Officer |
|---|---|
| Japanese Patent Office | |

Form PCT/ISA/210 (second sheet) (January 1985)